# EUROPEAN PATENT APPLICATION

(11) **EP 4 437 966 A1**
(43) Date of publication of application: **02.10.2024**
(21) Application number: 23164370.1
(22) Date of filing: 27.03.2023
(51) Int. Cl.: A61B 6/00, G06T 7/00

(54) **DYNAMIC IMAGE ACQUISITION AND STITCHING**

(71) Applicant: Koninklijke Philips N.V., 5656 AG Eindhoven (NL)
(72) Inventor: SAALBACH, Axel, Eindhoven (NL); SCHULZ, Heinrich, Eindhoven (NL); RENISCH, Steffen, 5656AG Eindhoven (NL); JOCKEL, Sascha Andreas, Eindhoven (NL)
(74) Representative: Philips Intellectual Property & Standards

(57) **Abstract**

The present invention relates to a computer-implemented method of acquiring and combining medical images of a subject. A first medical image of a subject acquired by a medical imaging device with a first field of view is analyzed with respect to an encompassment of a region of interest of the subject. In case the region of interest of the subject is not fully encompassed in the first medical image, at least one second field of view of the medical imaging device is determined, and data to instruct the medical imaging device to acquire at least one second medical image of the subject with the at least one second field of view are generated. The at least one second medical image encompasses at least the part of the region of interest of the subject that is not encompassed in the first medical image. Further, the generated data are transmitted to the medical imaging device, and the at least one second medical image of the subject acquired with the at least one second field of view is received and combined with the first medical image to a combined medical image. The combined medical image encompasses the region of interest of the subject, and is providing to a user.

## Description

### FIELD OF THE INVENTION

The present invention relates to a computer-implemented method of acquiring and combining medical images of a subject, a medical imaging system, a data processing apparatus, a computer program, and a computer-readable storage medium.

### BACKGROUND OF THE INVENTION

In conventional X-Ray imaging and fluoroscopy applications, acquisitions are typically acquired in a static setting, i.e., with a fixed system geometry and collimator configuration. For conventional chest posterior-anterior X-Ray imaging, guidelines exist which describe the optimal positioning of the patient and the collimator settings in order to ensure an optimal acquisition. However, due to variability in the patient anatomy or incidental finding, even carefully planned acquisitions could result in a sub-optimal imaging area. The implications range from unnecessary dose exposure, the need for re-takes up to an erroneous diagnosis. While fluoroscopy applications suffer from similar limitations, they include an additional, temporal, component. For instance, in swallowing exams, contrast medium exams, catheterizations, etc., the region of interest may change dynamically during the examination according to e.g., the passage of a contrast agent through a specific organ. To this end, either fixed image settings are employed, or manual interaction may be required.

As a result of the static system configuration, X-Ray imaging and fluoroscopy often result in a sub-optimal imaging area and unnecessary dose exposure. At the same time, modern collimators allow for the acquisition of even very small structures by limiting X-ray exposure to a specific region of interest.

The inventors of the present invention have thus found that it would be advantageous to have a method that solve these problems of sub-optimal imaging area and unnecessary dose exposure, while providing high-quality images that allow valuable diagnoses.

### SUMMARY OF THE INVENTION

It is an object of the present invention to provide an improved method of acquiring and combining medical images of a subject that restricts the imaged area to the necessary body or organ regions and reduces irradiation of tissue outside the region of interest.

The object of the present invention is solved by the subject matter of the independent claims, wherein further embodiments are incorporated in the dependent claims.

The described embodiments similarly pertain to the computer-implemented method of acquiring and combining medical images of a subject, the medical imaging system, the data processing apparatus, the computer program, and the computer-readable storage medium. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a computer-implemented method of acquiring and combining medical images of a subject. The method comprises the steps of receiving a first medical image of a subject acquired by a medical imaging device with a first field of view, analysing the first medical image with respect to an encompassment of a region of interest of the subject in the first medical image, and determining, in case the region of interest of the subject is not fully encompassed in the first medical image, at least one second field of view of the medical imaging device. The method comprises further the steps of generating data to instruct the medical imaging device to acquire at least one second medical image of the subject with the at least one second field of view, the at least one second medical image encompassing at least the part of the region of interest of the subject that is not encompassed in the first medical image, and transmitting the generated data to the medical imaging device. The method comprises further the steps of receiving the at least one second medical image of the subject acquired by the medical imaging device with the at least one second field of view, combining the first medical image and the at least one second medical image to a combined medical image, the combined medical image encompassing the region of interest of the subject, and providing the combined medical image.

Thus, a dynamic acquisition scheme is proposed that uses preferably an AI-based proposal system to determine the need for acquiring at least one additional image and the respective imaging region in combination with an automated X-ray or fluoroscopy system that is able to automatically adjust and correct the field of view to be imaged. The ability to change the field of view during the acquisition based on the already acquired image data offers promising options to restrict the imaged area to the strictly necessary body or organ regions, to dynamically extend the area in the presence of imaging artifacts or incidental findings, and to reduce or even avoid irradiation of tissue outside the region of interest.

Preferably, the first and second image, respectively, can comprise a plurality of images. In particular, when using, for example, a time series of images as an input, the first medical image can comprise multiple input images. A time series of images can be understood as a sequence of images taken at successive equally spaced points in time. Each of these images can be analyzed with respect to the encompassed imaging area, and for each image of the plurality of images, one or more second images can be acquired to correct for the desired imaging area or field of view. In case of a time series, each of the plurality of input images can be corrected by the method of the present invention before a subsequent image of the time series is acquired. However, in case a previous image of a time series is corrected and extended based on the proposed method, the proposed field of view can also be considered when acquiring a subsequent image of the time series.

Specifically for tracking applications, the first acquired image may not encompass the region of interest and multiple iterations of the inventive method may be necessary in order to cover the region of interest. In case of a fluoroscopy application, the temporal information could provide insights into the speed of the distribution of a contrast agent, for example, in the body, and hence could provide a better prediction of a diagnosis. The subsequent images of a time series of images may be encoded as individual channels in the input image, for example, and the image may be analyzed using the method according to the invention as described previously.

In an embodiment of the invention, the medical imaging device is an X-ray imaging device or a fluoroscopy device.

In an embodiment of the invention, the generated data to instruct the medical imaging device to acquire at least one second medical image comprise instructions to change the field of view of the medical imaging device by changing a position and/or orientation of a radiation source and/or of a radiation detector of the medical imaging device.

In an embodiment of the invention, the generated data to instruct the medical imaging device to acquire at least one second medical image comprise instructions to change the field of view of the medical imaging device by changing a configuration of a collimator of the medical imaging device.

In an embodiment of the invention, the medical imaging system comprises a multi-leaf collimator.

In an embodiment of the invention, the generated data comprises a location, a size, and/or a shape of the second field of view.

In an embodiment of the invention, the steps of analysing the first medical image and/or determining at least one second field of view of the medical imaging device are performed by a trained artificial intelligence.

In an embodiment of the invention, the artificial intelligence comprises a regressor network or a region proposal network.

In an embodiment of the invention, the region of interest comprises an anatomical structure, a spreading of a contrast agent, a clinical finding, or a device inserted into the subject.

In an embodiment of the invention, the first field of view of the first image is derived from a camera image or an X-ray pre-shot of the subject.

In an embodiment of the invention, the first medical image of the subject comprises a plurality of first medical images of the subject taken at successive points in time, and the step of determining at least one second field of view of the medical imaging device comprises analysing a temporal change of the region of interest in the plurality of first medical images of the subject.

In an embodiment of the invention, the method comprises the step of proposing the acquisition of the at least one second medical image with the at least one second field of view to a user.

According to another aspect of the invention, there is provided a data processing apparatus comprising a processor configured to perform the steps of the method according to any of the preceding embodiments.

According to another aspect of the invention, there is provided a medical imaging system comprising the data processing apparatus according to the preceding embodiment.

According to another aspect of the invention, there is provided a computer program comprising instructions which, when the program is executed by a computer, cause the computer to carry out the steps of the method according to any of the preceding embodiments. The computer program can be performed on one or more processing units, which are instructed to perform the method according to any of the preceding embodiments.

According to another aspect of the invention, there is provided a computer-readable storage medium comprising instructions which, when executed by a computer, cause the computer to carry out the steps of the method according to any of the preceding embodiments.

Thus, the benefits provided by any of the previously cited aspects equally apply to all of the other aspects and vice versa.

In summary, the invention relates to a computer-implemented method of acquiring and combining medical images of a subject. A first medical image of a subject acquired by a medical imaging device with a first field of view is analyzed with respect to an encompassment of a region of interest of the subject. In case the region of interest of the subject is not fully encompassed in the first medical image, at least one second field of view of the medical imaging device is determined, and data to instruct the medical imaging device to acquire at least one second medical image of the subject with the at least one second field of view are generated. The at least one second medical image encompasses at least the part of the region of interest of the subject that is not encompassed in the first medical image. Further, the generated data are transmitted to the medical imaging device, and the at least one second medical image of the subject acquired with the at least one second field of view is received and combined with the first medical image to a combined medical image. The combined medical image encompasses the region of interest of the subject, and is providing to a user.

One of the advantages of embodiments of the present invention to change the field of view during the acquisition based on the already acquired image data may be to restrict the imaged area to the strictly necessary body or organ regions. Another advantage may be that the imaged area can be dynamically extended in the presence of imaging artifacts or incidental findings. Another advantage may be that irradiation of tissue outside the region of interest can be reduced or even avoided.

These advantages are non-limiting and other advantages may be envisioned within the context of the present application.

These aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a block diagram of a computer-implemented method of acquiring and combining medical images of a subject according to an embodiment of the invention.
Fig. 2 shows a schematic setup of a processing unit and a medical imaging system carrying out the steps of the method according to an embodiment of the invention.

### DETAILED DESCRIPTION OF EMBODIMENTS

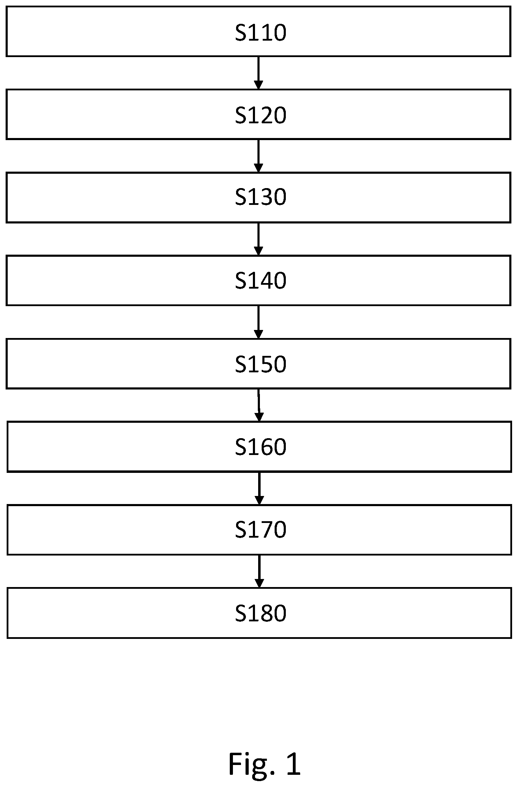

With reference to Fig. 1, this shows a block diagram of a computer-implemented method of acquiring and combining medical images of a subject 110 according to an embodiment of the invention. The method comprises the step S110 of receiving a first medical image 120 of a subject 110 acquired by a medical imaging device 100 with a first field of view 121, the step S120 of analysing the first medical image 120 with respect to an encompassment of a region of interest 140 of the subject 110 in the first medical image 120, and the step S130 of determining, in case the region of interest 140 of the subject 110 is not fully encompassed in the first medical image 120, at least one second field of view 131 of the medical imaging device 100. The method comprises further the step S140 of generating data 160 to instruct the medical imaging device 100 to acquire at least one second medical image 130 of the subject 110 with the at least one second field of view 131, the at least one second medical image 130 encompassing at least the part of the region of interest 140 of the subject 110 that is not encompassed in the first medical image 120, and the step S150 of transmitting the generated data 160 to the medical imaging device 100. The method comprises further the step S160 of receiving the at least one second medical image 130 of the subject 110 acquired by the medical imaging device 110 with the at least one second field of view 131, the step S170 of combining the first medical image 120 and the at least one second medical image 130 to a combined medical image 150, the combined medical image 150 encompassing the region of interest 140 of the subject 110, and the step S180 of providing the combined medical image 150.

Thus, embodiments of the present invention propose a method using a dynamic acquisition scheme with preferably an AI-based proposal system to determine the need for acquiring at least one additional image and the respective imaging region in combination with an automated X-ray or fluoroscopy system that is able to automatically adjust and correct the field of view to be imaged. The ability to change the field of view during the acquisition based on the already acquired image data offers promising options to restrict the imaged area to the strictly necessary body or organ regions, to dynamically extend the area in the presence of imaging artifacts or incidental findings, and/or to reduce or even avoid irradiation of tissue outside the region of interest.

The method according to the invention may use two main components: A processing unit 200 which analyses the already acquired image data of a first medical image 120 and predicts the need for an additional acquisition, its location, and/or expected dimensions, and a medical imaging device 100 that may allow for dynamic acquisition of additional scans with adapted collimation and/or positioning and/or orientation of parts of the medical imaging device 100. In embodiments, the medical imaging device 100 is an X-ray imaging device or a fluoroscopy device. Especially in X-ray applications, it may be necessary to reduce the radiation exposure to the patient. Thus, the field of view of the imaging device 100 can be advantageously limited to a very small imaging region to avoid excessive exposure. For adjusting the field of view, collimators 170 may be used to spatially restrict and confine the radiation beam.

For the X-ray or fluoroscopy system, different options can be utilized, like a system designed to collimate symmetrically around the central X-ray beam, or a system comprising a flexible collimator configuration.

Accordingly, in embodiments, the generated data 160 to instruct the medical imaging device 100 to acquire at least one second medical image 130 comprise instructions to change the field of view of the medical imaging device 100 by changing a position and/or orientation of a radiation source and/or of a radiation detector of the medical imaging device 100. As many X-ray systems are designed to collimate symmetrically around the central X-ray beam, the configuration of the imaging system 100, for example tube and detector position, can be automatically adjusted based on the needs for acquiring the one or more additional images. Thus, the use of an automated imaging system may be proposed that automatically adjusts the configuration of the system with regard to the position of the tube and detector based on the outcome of the analysis of the already required image data like the first medical image 120.

Additionally or alternatively, in embodiments, the generated data 160 to instruct the medical imaging device 100 to acquire at least one second medical image 120 comprise instructions to change the field of view of the medical imaging device 100 by changing a configuration of a collimator 170 of the medical imaging device 100. Thus, an X-ray system with a flexible collimator configuration can be utilized, where the system geometry of X-ray tube and detector can optionally remain unchanged, while the flexible collimator 170 allows for the acquisition of a specific region of interest with the medical imaging device 100. As in practical applications, the utilized field of view of an imaging device 100 may be a result of the position of the source, the detector and the collimator 170, in combination with the patient 110, it should be understood that none of the parameters defined before defines the field of view alone.

In embodiments, the medical imaging system 100 comprises a multi-leaf collimator as collimator 170. A multi-leaf collimator can be understood as beam-limiting device comprising a plurality of individual leaves of a radiation absorbing material, which can move independently in and out of the path of the radiation beam in order to shape and/or vary the intensity of the radiation beam.

In embodiments, the generated data 160 comprises a location, a size, and/or a shape of the second field of view 131. Particularly in case of a multi-leaf collimator, the field of view can be very complex and not restricted to rectangular shapes, for example. Furthermore, depending on the determined at least one second field of view, the source, the detector and/or the collimator might need to be adapted in order to acquire a second image.

In embodiments, the steps of analysing the first medical image 120 and/or determining at least one second field of view 131 of the medical imaging device 100 are performed by a trained artificial intelligence. Thus, a recommender engine can be used, which predicts the need and parameters of a subsequent image acquisition. To this end, different artificial intelligence (AI) algorithms can be considered. All of them are assumed to generate at least two different types of output. A first output indicates whether an additional acquisition needs to be acquired in order to image the complete region of interest of the patient. A second output defines the system configuration for the additional acquisition. This can include the configuration of a collimator 170 as well as other information regarding the system geometry, e.g., tube and detector position. For the training and evaluation of the artificial intelligence algorithm, the process of a sequential acquisition can be simulated by extracting parts from existing acquisitions to simulate an incomplete image of a region of interest.

In embodiments, the artificial intelligence comprises a regressor network or a region proposal network. For the training and evaluation, potential embodiments may include a regressor network, a region proposal network or conventional computer vision and machine learning techniques. A regressor network can be understood as an AI system with a deep learning backbone comprising a convolutional neural network, which may be extended by a fully connected layer for the prediction of different outputs, given an input image. A region proposal network may be employed in computer vision applications to detect objects. Therefore, different image regions are evaluated in sliding window like fashion, while a proposal layer predicts candidate locations which can be later integrated into individual predictions. Similar to the regressor network, this concept can be extended to predict multiple candidate configurations for the subsequent acquisition which are then integrated. Using combinations of conventional computer vision and machine learning techniques may result in solutions similar to the regression network approach. Specifically, a combination of image procession features can be evaluated on sub-region followed by the application of machine learning techniques like support vector regression or decision trees.

In embodiments, the region of interest comprises an anatomical structure, a spreading of a contrast agent, a clinical finding, and/or a device inserted into the subject. Based on the initial image, the proposed method and system can be used to track anatomical structures or devices with minimal dose exposure for the surrounding tissue.

In embodiments, the first field of view 121 of the first image 120 is derived from a camera image or an X-ray pre-shot of the subject 110. Thus, the initial acquisition of the first image 120 can be guided by means of a (RGBD) camera system or a similar sensor, or an X-ray pre-shot.

In embodiments, the first medical image of the subject comprises a plurality of first medical images of the subject taken at successive points in time, and the step of determining at least one second field of view of the medical imaging device comprises analysing a temporal change of the region of interest in the plurality of first medical images of the subject.

Thus, in a dynamic environment of, for example, a fluoroscopy examination, and in order to predict good control parameters for the medical imaging system to acquire the at least one second medical image, additional images from previous time-points of, for example, a time series of images can be provided and used in order to facilitate, e.g., velocity estimations of contrast agents.

In embodiments, the method comprises the step of proposing the acquisition of the at least one second medical image 130 with the at least one second field of view 131 to a user. In addition or alternatively, in case of detected incidental findings, the proposed method and system may automatically propose to adjust the acquired field of view. In case of fluoroscopy acquisitions, the system can either propose an additional scan region or it can increase the region gradually, thus allowing a cinematic view on the entire region of interest.

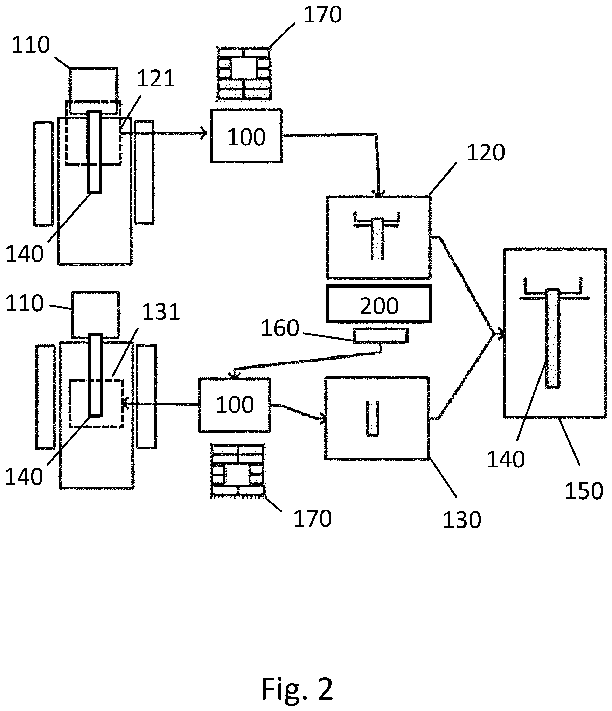

With reference to Fig. 2, and continuing reference to Fig. 1, a schematic setup of a processing unit 200 and a medical imaging system 100 carrying out the steps of the method according to an embodiment of the invention is shown in Fig. 2. At least a part of a subject 110 is imaged with a medical imaging device 100 with a first setting of a collimator 170, thus resulting in a first field of view 121 of the medical imaging device 100. The obtained first medical image 120 covers at least a part of a region of interest 140 of the subject 110. A processing unit 200 configured for carrying out the steps of the method according to an embodiment of the invention analyses the first medical image 120 and decides whether the region of interest 140 is fully depicted in the first medical image 120. In case the region of interest 140 is not fully depicted in the first medical image 120, data 150 are generated in order to instruct the medical imaging device 100 to acquire at least a second medical image 130 with a second field of view 131 of the subject 110 with the region of interest 140. The second field of view 131 can be dependent on a setting of the collimator 170. The second medical image 130 is combined, for example by stitching, with the first medical image 120 to generate a combined medical image 150, which fully encompasses the region of interest 140.

After acquisition of the one or more additional images second medical images 130 with the automated medical imaging system 100 according to the field of view determined by analysing the first medical image 120, the one or more second medical images 130 are combined with the first medical image 120 to a single, combined medical image 150. For example by stitching the at least one second image with the first image, an image can be generated, which favorably encompasses the complete region of interest of the subject 110.

Therefore, given an initial X-Ray or fluoroscopic image, the method of the present invention can be employed to predict for, and location of an additional image acquisition of a second medical image 130 in addition to the existing image data of the first medical image 120. This additional image acquisition can be outside of the range of the existing image data and/or detector limits, and can be acquired, for example, by changing a collimator configuration and/or by changing the setup of the X-ray tube and X-ray detector.

Preferably, the first image 120 and second image 130, respectively, can comprise a plurality of images. In particular, when using a time series as an input, the first medical image 120 can comprise multiple input images. Each of these images can be analyzed with respect to the encompassed imaging area, and for each image of the plurality of images, one or more second images 120 can be acquired to correct for the desired imaging area or field of view.

Specifically for tracking applications, the first acquired image may not encompass the region of interest and multiple iterations of the inventive method may be necessary in order to cover the region of interest.

According to another aspect of the invention, there is provided a data processing apparatus 200 comprising a processor configured to perform the steps of the method according to any of the preceding embodiments.

According to another aspect of the invention, there is provided a medical imaging system 100 comprising the data processing apparatus 200 according to the preceding embodiment.

According to another aspect of the invention, there is provided a computer program comprising instructions which, when the program is executed by a computer, cause the computer to carry out the steps of the method according to any of the preceding embodiments. The computer program can be performed on one or more processing units, which are instructed to perform the method according to any of the preceding embodiments.

According to another aspect of the invention, there is provided a computer-readable storage medium comprising instructions which, when executed by a computer, cause the computer to carry out the steps of the method according to any of the preceding embodiments.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: medical imaging device
- 110: subject
- 120: first medical image
- 121: first field of view
- 130: second medical image
- 131: second field of view
- 140: region of interest
- 150: combined medical image
- 160: generated data
- 170: collimator
- 200: data processing apparatus

## Claims

1. A computer-implemented method of acquiring and combining medical images of a subject (110), the method comprising the steps of:
receiving a first medical image (120) of a subject (110) acquired by a medical imaging device (100) with a first field of view (121);
analysing the first medical image (120) with respect to an encompassment of a region of interest (140) of the subject (110) in the first medical image (120);
determining, in case the region of interest (140) of the subject (110) is not fully encompassed in the first medical image (120), at least one second field of view (131) of the medical imaging device (100);
generating data (160) to instruct the medical imaging device (100) to acquire at least one second medical image (130) of the subject (110) with the at least one second field of view (131), the at least one second medical image (130) encompassing at least the part of the region of interest (140) of the subject (110) that is not encompassed in the first medical image (120);
transmitting the generated data (160) to the medical imaging device (100);
receiving the at least one second medical image (130) of the subject (110) acquired by the medical imaging device (110) with the at least one second field of view (131);
combining the first medical image (120) and the at least one second medical image (130) to a combined medical image (150), the combined medical image (150) encompassing the region of interest (140) of the subject (110); and
providing the combined medical image (150).

2. The method according to claim 1, wherein the medical imaging device (100) is an X-ray imaging device or a fluoroscopy device.

3. The method according to any of claims 1 or 2, wherein the generated data (160) to instruct the medical imaging device (100) to acquire at least one second medical image (130) comprise instructions to change the field of view of the medical imaging device (100) by changing a position and/or orientation of a radiation source and/or of a radiation detector of the medical imaging device (100).

4. The method according to any of the preceding claims, wherein the generated data (160) to instruct the medical imaging device (100) to acquire at least one second medical image (130) comprise instructions to change the field of view of the medical imaging device (100) by changing a configuration of a collimator (170) of the medical imaging device.

5. The method according to claim 4, wherein the medical imaging system (100) comprises a multi-leaf collimator (170).

6. The method according to any of the preceding claims, wherein the generated data (160) comprises a location, a size, and/or a shape of the at least one second field of view (131).

7. The method according to any of the preceding claims, wherein the steps of analysing the first medical image (120) and/or determining at least one second field of view (131) of the medical imaging device (100) are performed by a trained artificial intelligence.

8. The method according to claim 7, wherein the artificial intelligence comprises a regressor network or a region proposal network.

9. The method according to any of the preceding claims, wherein the region of interest (140) comprises an anatomical structure, a spreading of a contrast agent, a clinical finding, or a device inserted into the subject (110).

10. The method according to any of the preceding claims, wherein the first medical image (120) of the subject (110) comprises a plurality of first medical images of the subject (110) taken at successive points in time, and wherein the step of determining at least one second field of view (131) of the medical imaging device (100) comprises analysing a temporal change of the region of interest in the plurality of first medical images of the subject (110).

11. The method according to any of the preceding claims, wherein the method comprises the step of proposing the acquisition of the at least one second medical image (130) with the at least one second field of view (131) to a user.

12. A data processing apparatus (200) comprising means for carrying out the steps of the method according to any of claims 1 to 11.

13. A medical imaging system comprising the data processing apparatus (200) according to claim 12.

14. A computer program comprising instructions which, when the program is executed by a computer, cause the computer to carry out the steps of the method according to any of claims 1 to 11.

15. A computer-readable storage medium comprising instructions which, when executed by a computer, cause the computer to carry out the steps of the method according to any of claims 1 to 11.
